# EUROPÄISCHE PATENTSCHRIFT

(11) **EP 1 363 594 B1**
(45) Veröffentlichungstag und Bekanntmachung des Hinweises auf die Patenterteilung: **21.11.2007**
(21) Anmeldenummer: 02710842.2
(22) Anmeldetag: 06.02.2002
(51) Int. Cl.: A61K 8/49, A23L 1/30, C07H 17/07

(54) **KOSMETISCHE FORMULIERUNG ENTHALTEND FLAVONOID-DERIVATE**
COSMETIC FORMULATIONS CONTAINING FLAVONOID DERIVATIVES
FORMULATIONS COSMETIQUES RENFERMANT DES DERIVES DE FLAVONOIDES

(30) Priorität: 02.03.2001 DE 10110105
(43) Veröffentlichungstag der Anmeldung: 26.11.2003
(60) Teilanmeldung: 07015474.5
(73) Patentinhaber: Merck Patent GmbH, 64293 Darmstadt (DE)
(72) Erfinder: WIRTH, Corinna, 64283 Darmstadt (DE); BUCHHOLZ, Herwig, 60599 Frankfurt (DE); CAROLA, Christophe, 63225 Langen (DE)
(86) Internationale Anmeldenummer: PCT/EP2002/001200
(87) Internationale Veröffentlichungsnummer: WO 2002/069926

(56) Entgegenhaltungen:
- EP-A- 0 420 376
- DE-A- 19 631 222
- DE-A- 19 922 287
- US-A- 5 034 213
- DATABASE CA [Online] CHEMICAL ABSTRACTS SERVICE, COLUMBUS, OHIO, US; OKAMURA, HIDEO ET AL: "Antioxidative novel flavonoid glycoside of Eucalyptus" retrieved from STN Database accession no. 121:141654 XP002194814 & JP 06 100584 A (JUMOKU CHUSHUTSU SEIBUN RYO, JAPAN) 12. April 1994 (1994-04-12)
- JUNGBLUT, T.P. ET AL.: "STRUCTURES OF UV-B INDUCED SUNSCREEN PIGMENTS OF THE SCOTS PINE (PINUS SYLVESTRIS L.)" ANGEW. CHEM. INT. ED. ENGL., Bd. 34, Nr. 3, 1995, Seiten 312-314, XP001064556

## Beschreibung

Die Erfindung betrifft die Verwendung von Flavonoid-Derivaten in kosmetischen Formulierungen, sowie neue UV-aktive Verbindungen.

Eine mehr oder minder stark ausgeprägte Sonnenbräune der Haut gilt in der modernen Gesellschaft als attraktiv und als Ausdruck von Dynamik und Sportlichkeit. Neben dieser erwünschten Wirkung der Sonne auf die Haut treten eine Reihe von unerwünschten Nebenwirkungen auf, wie Sonnenbrand oder vorzeitige Hautalterung und Faltenbildung. Inzwischen sind eine Anzahl von leistungsfähigen UV-Filtern entwickelt worden, die in Form von Cremes, Lotionen oder Gelen auf die Haut aufgetragen auch bei stärkerer Sonneneinwirkung die Entwicklung von Sonnenbrand wirksam verzögern können. Der in der pharmazeutischen oder kosmetischen Zubereitung enthaltene UV-Filter bildet auf der Oberfläche der Haut einen Film bzw. eine Schicht aus und dringt nicht mit weiteren in der Zubereitung enthaltenen pflegenden Substanzen in tiefere Hautschichten vor. Bekannte UV-Filter bzw. Sonnenschutzmittel wirken also in der Weise, dass sie bestimmte Bereiche des Sonnenlichts absorbieren und somit diese Strahlung nicht in tiefere Schichten der Haut vordringen kann. Bekanntlich wird der gefährlichste Teil der Sonnenstrahlung von den ultravioletten Strahlen mit einer Wellenlänge von weniger als 400 nm gebildet. Die untere Grenze der ultravioletten Strahlen, welche die Erdoberfläche erreichen, ist durch die Absorption in der Ozonschicht auf ca. 280 nm beschränkt. Die heute in der Kosmetik üblichen Sonnenschutzfilter absorbieren in einem Wellenlängenbereich von 280 bis 400 nm. Dieser Bereich umfasst UV-B-Strahlen mit einer Wellenlänge zwischen 280 und 320 nm, die bei der Bildung eines Sonnenerythems eine entscheidende Rolle spielen, wie auch UV-A-Strahlen, mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische und irritative Reaktionen auslösen können.

Hautschädigungen werden nicht nur durch Sonnenlicht verursacht, sondern auch durch andere äußere Einflüsse, wie Kälte oder Wärme. Ferner unterliegt die Haut einer natürlichen Alterung, wodurch Falten entstehen und die Spannkraft der Haut nachlässt.

Aufgabe pflegender Kosmetik ist es, nach Möglichkeit den Eindruck einer jugendlichen Haut zu erhalten. Prinzipiell stehen verschiedene Wege offen, um dieses Ziel zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen. Die Idee ist also, vorbeugend einzugreifen und dadurch den Alterungsprozess hinauszuzögern. Ein Beispiel hierfür sind die bereits erwähnten UV-Filter, welche durch Absorption bestimmter Wellenlängenbereiche eine Schädigung der Haut vermeiden oder zumindest vermindern. Entsprechend der Lage ihrer Absorptionsmaxima werden UV-Absorber für kosmetische und dermatologische Zubereitungen in UV-A- und UV-B-Absorber eingeteilt, wobei UV-A-Absorber üblicherweise auch im UV-B-Bereich absorbieren und daher alternativ auch als Breitbandabsorber oder -filter bezeichnet werden.

Die bekannten UV-Filter haben jedoch oftmals Nachteile: beispielsweise ist ihre Hautverträglichkeit nicht befriedigend oder ihre Absorptionseigenschaften sind ungenügend. Die ungenügenden Absorptionseigenschaften können sich z.B. darin äußern, dass nur ein geringer Teil des UV-Spektrums absorbiert wird oder dass der Absorptionskoeffizient bei einer gegebenen Wellenlänge nicht befriedigend ist.

Während bei UV-Filtern das schädigende Ereignis, die UV-Strahlung, von der Haut abgeschirmt wird, versucht man bei einem weiteren Weg, die natürlichen Abwehr- bzw. Reparaturmechanismen der Haut gegen das schädigende Ereignis zu unterstützen. Schliesslich verfolgt man als weiteren Ansatzpunkt die mit zunehmendem Alter sich abschwächenden Abwehrfunktionen der Haut gegen schädigende Einflüsse auszugleichen, indem Substanzen von aussen zugeführt werden, die diese nachlassende Abwehr- bzw. Reparaturfunktion ersetzen können. Beispielsweise besitzt die Haut die Fähigkeit, Radikale, die durch äussere oder innere Stressfaktoren erzeugt werden, abzufangen. Diese Fähigkeit schwächt sich mit zunehmendem Alter ab, wodurch sich der Alterungsprozess mit zunehmendem Alter beschleunigt.

Eine weitere Schwierigkeit bei der Herstellung von Kosmetika besteht darin, dass Wirkstoffe, die in kosmetische Formulierungen eingearbeitet werden sollen, oftmals nicht stabil sind und in der Formulierung geschädigt werden können. Die Schädigungen können beispielsweise durch eine Reaktion mit Luftsauerstoff oder durch die Absorption von UV-Strahlen verursacht werden. Die so geschädigten Moleküle können durch ihre Strukturänderung z.B. ihre Farbe ändern und/oder ihre Wirksamkeit verlieren.

Aufgabe der vorliegenden Erfindung war es daher, kosmetische Formulierungen zur Verfügung zu stellen, die die Nachteile des Standes der Technik vermeiden und insbesondere vorteilhafte Absorptionseigenschaften besitzen.

Überraschend wurde nun gefunden, dass diese Aufgabe durch die Verwendung gemäß Anspruch 1 gelöst wird.

Verbindungen der Formel IA sind bereits bekannt. Allerdings wurden diese bisher nicht in kosmetischen Formulierungen verwendet.

Eine bekannte Verbindung der Formel IA ist z.B. Kaempferol 3-(6''-p-coumarylglucosid), welches auch als Tilirosid bezeichnet wird.

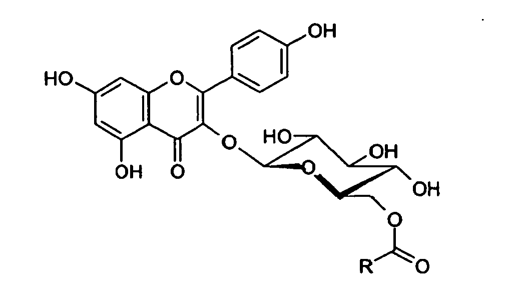

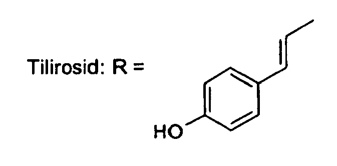

In der DE 195 44 905 A1 wird z.B. ein Verfahren zur Herstellung von Pflanzenextrakten enthaltend Tilirosid beschrieben sowie die Verwendung der Pflanzenextrakte in Arzneimitteln und Lebensmittelprodukten. Kosmetische Formulierungen enthaltend Tilirosid werden in der DE 195 44 905 A1 jedoch nicht beschrieben.

In der DE 199 22 287 A1 wird Tilirosid als ein Ausgangsflavonoid zur Herstellung von Tilirosidestern, deren Säureeinheit 3 bis 30 C-Atome enthält, beschrieben. Diese Ester werden in Kosmetika verwendet. In der DE 199 22 287 A1 werden jedoch keine kosmetischen Formulierungen enthaltend Tilirosid beschrieben.

Die erfindungsgemäßen kosmetischen Formulierungen enthaltend eine oder mehrere Verbindungen der Formel IA besitzen beispielsweise den Vorteil, dass sie sowohl im UV-A- als auch im UV-B-Bereich absorbieren. Dies bedeutet, dass durch den Einsatz der erfindungsgemäßen Formulierungen ein Breitband-UV-Schutz erreicht werden kann. Zudem weisen die erfindungsgemäßen Formulierungen gute Absorptionskoeffizienten auf. Dies wird im folgenden am Beispiel der Substanz Tilirosid verdeutlicht.

Tilirosid absorbiert im UV-B-Bereich, also in dem Wellenlängenbereich zwischen 290 und 320 nm, mit einem Absorptionsmaximum bei λₘₐₓ = 316 nm und zusätzlich im UV-A-Bereich, also in dem Wellenlängenbereich zwischen 320 und 400 nm, mit einer Absorptionsschulter bei λₛₕ = 349 nm. Der Absorpionskoeffizient ε bei λₘₐₓ = 316 nm ist ε = 23260 und bei λₛₕ = 349 nm ist ε = 13500.

Die vorteilhaften UV-absorbierenden Eigenschaften der in den erfindungsgemäßen kosmetischen Formulierungen enthaltenen Verbindungen der Formel IA werden insbesondere deutlich, wenn man sie mit denen anderer Substanzen vergleicht, die im Handel erhältlich sind und in Sonnenschutzformulierungen verwendet werden. Beispielsweise zeigt Eusolex^{®} 6300 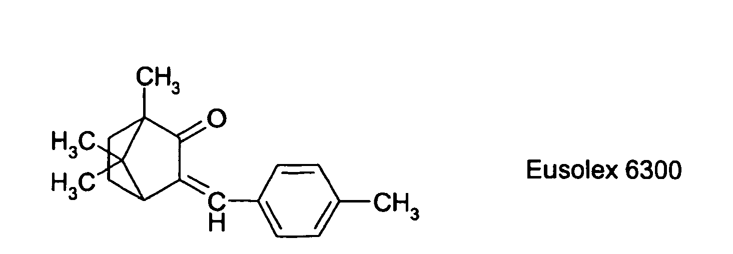 im UV-B-Bereich ein Absorptionsmaximum bei λₘₐₓ = 300 nm. Der Absorptionskoeffizient bei dieser Wellenlänge ist 23420. Eusolex^{®} 6300 absorbiert jedoch nicht im UV-A-Bereich. Dies bedeutet, dass Tilirosid und Eusolex^{®} 6300 im UV-B-Bereich einen vergleichbaren Absorptionskoeffizienten besitzen, während Tilirosid im UVA-Bereich signifikant bessere Absorptionseigenschaften aufweist.

Gegenstand der vorliegenden Erfindung ist auch die Verwendung von einer oder mehreren Verbindungen der Formel IA als UV-Filter in kosmetischen Formulierungen.

Neben den vorteilhaften UV-absorbierenden Eigenschaften besitzen die Verbindungen der Formel IA zudem vorteilhafte antioxidierende und radikalfangende Eigenschaften. Weiterhin Gegenstand der vorliegenden Erfindung ist somit auch die Verwendung von einer oder mehreren Verbindungen der Formel IA als Radikalfänger und/oder Antioxidans in kosmetischen Formulierungen.

Durch ihre Wirkung als Antioxidans wirken Verbindungen der Formel IA auch stabilisierend auf Formulierungen, die z.B. in der Kosmetik verwendet werden. Durch Beigabe von Verbindungen der Formel IA zu den entsprechenden Produkten bleiben diese daher länger stabil und verändern ihr Aussehen nicht. Insbesondere bleibt auch bei länger dauernder Anwendung bzw. längerer Lagerung die Wirksamkeit der Inhaltsstoffe erhalten. Dies ist besonders vorteilhaft bei Sonnenschutzmitteln, da diese Kosmetika besonders hohen Belastungen durch UV-Strahlen ausgesetzt sind.

Die Formulierungen enthaltend eine oder mehrere Verbindungen der Formel IA eignen sich besonders zum Schutz menschlicher Haut bzw. zum Schutz von Körperzellen gegen oxidativen Stress, d.h. z.B. gegen Schädigungen durch Radikale, wie sie z.B. durch Sonneneinstrahlung, Wärme oder andere Einflüsse erzeugt werden. Die Formulierungen enthaltend eine oder mehrere Verbindungen der Formel IA sind insbesondere zur Verringerung der Hautalterung geeignet.

Gegenstand der vorliegenden Erfindung ist somit auch die Verwendung von einer oder mehreren Verbindungen der Formel IA als Wirkstoff zum Schutz gegen oxidativen Stress in kosmetischen Formulierungen. Ein weiterer Gegenstand der vorliegenden Erfindung ist zudem die Verwendung von einer oder mehreren Verbindungen der Formel IA zur Vermeidung der Hautalterung in kosmetischen Formulierungen.

Die Verbindungen der Formel IA besitzen zudem anti-allergische, anti-inflammatorische, entzündungshemmende und anti-irritative Eigenschaften und können somit zur Behandlung oder vorbeugenden Behandlung von Allergien, Entzündungen und Irritationen, insbesondere der Haut, verwendet werden. Ein weiterer Gegenstand der vorliegenden Erfindung ist daher die Verwendung von einer oder mehreren Verbindungen der Formel IA als Wirkstoff mit anti-allergischer, anti-inflammatorischer, entzündungshemmender und anti-irritativer Wirkung in kosmetischen Formulierungen.

Desweiteren besitzen Verbindungen der Formel IA wie z.B. Tilirosid nur eine schwach ausgeprägte Eigenfarbe. Die schwach ausgeprägte Eigenfarbe ist z.B. dann von großem Vorteil, wenn in den Produkten eine Eigenfarbe der Inhaltsstoffe aus ästhetischen Gründen unerwünscht ist.

In den Verbindungen der Formel IA sind die Alkoxygruppen vorzugsweise linear und besitzen 1 bis 8 C-Atome. Diese Gruppen entsprechen somit den Formeln -O-(CH₂)ₘ-H, wobei m 1, 2, 3, 4, 5, 6, 7 oder 8 und insbesondere 1 bis 5 bedeutet.

Falls einer oder mehrere der Reste R¹ bis R³ und R⁸ in den Verbindungen der Formel IA einen Mono- oder Oligoglykosidrest bedeuten, so ist dieser Glykosidrest über ein Sauerstoffatom direkt an den entsprechenden Benzolring in Formel IA gebunden. Die Mono- oder Oligoglykosidreste sind vorzugsweise aus 1 bis 3 Glykosideinheiten aufgebaut. Vorzugsweise sind diese Einheiten ausgewählt aus der Gruppe der Hexosylreste insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Die in dem Rest R⁴ der Verbindungen der Formel IA enthaltenen Mono- oder Diglykosidreste sind über ein Sauerstoffatom gebunden. Die bei den Resten R¹ bis R³ und R⁸ bevorzugten Einheiten sind auch für den in Rest R⁴ enthaltenen Mono- oder Diglykosidrest bevorzugt. Insbesondere bevorzugt ist der in dem Rest R⁴ enthaltene Mono- oder Diglykosidrest ausgewählt aus der Gruppe bestehend aus den Resten von Glucose, Rhamnose und Rutinose.

In einer bevorzugten Ausführungsform der Erfindung, insbesondere wenn die Wasserlöslichkeit der Verbindungen der Formel IA gesteigert werden soll, ist an eine oder an mehrere Hydroxygruppen der in den Substituenten R¹ bis R⁴ und R⁸ genannten Reste eine polare Gruppe gebunden, z.B. jeweils unabhängig voneinander eine Sulfat- oder Phosphatgruppe. Geeignete Gegenionen sind beispielsweise die Ionen der Alkali- oder Erdalkalimetalle, wobei diese z.B. aus Natrium oder Kalium ausgewählt sind.

In einer weiteren bevorzugten Ausführungsform der Erfindung sind diejenigen Verbindungen der Formel IA bevorzugt, in denen die in dem Substituenten R⁴ enthaltenen Reste mit aromatischer Komponente über eine Estergruppe -OOC- an den ebenfalls im Rest R⁴ enthaltenen Mono- oder Diglykosidrest gebunden sind.

In einer weiteren bevorzugten Ausführungsform der Erfindung leiten sich Teilformeln der Formel IA von den Verbindungen der nachstehenden Gruppe ab: Rutin, Trishydroxyethylrutin (Troxerutin), Isoquercetin, Trishydroxyethylisoquercetin (Troxeisoquercetin) und Astragalin sowie deren Sulfate und Phosphate.

In einer Ausführungsform sind die in den Formulierungen enthaltenen Verbindungen ausgewählt aus den Verbindungen der Formel IA 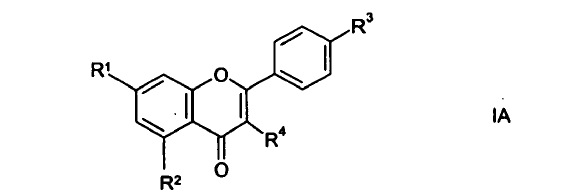 worin
- R¹, R² und R³: jeweils unabhängig voneinander OH, CH₃COO, einen Alkoxyrest mit 1 bis 8 C-Atomen oder einen Monoglykosidrest bedeuten,
- R⁴: ein Mono- oder Diglykosidrest ist, wobei an den Glykosidrest jeweils über eine Gruppe -O- 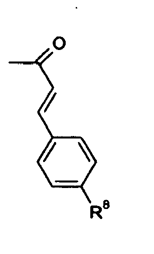 gebunden ist,
- R⁸: die Bedeutung der Reste R¹ bis R³ besitzt, und
worin ein oder mehrere Wasserstoffatome in den OH-Gruppen des oder der Glykosidreste jeweils unabhängig voneinander auch durch Acetyl oder durch Alkylreste mit 1 bis 8 C-Atomen ersetzt sein können und wobei an ein oder mehrere Hydroxygruppen der Verbindungen der Formel IA jeweils unabhängig voneinander auch Sulfat oder Phosphat gebunden sein kann.

In einer bevorzugten Ausführungsform ist der Rest R² in den Verbindungen der Formel IA ausgewählt aus OH, CH₃COO oder einem Alkoxyrest mit 1 bis 8 C-Atomen.

In den Verbindungen der Formel IA können alle OH-Gruppen des Mono- oder Diglykosidrests von R⁴ mit einer Gruppe der Formel 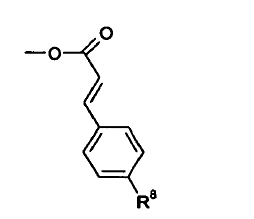 verestert sein. Vorzugsweise sind jedoch nur ein oder zwei der von diesem Rest abgeleiteten Reste an den Glykosidrest gebunden.

Wenn R⁴ ein Mono- oder Diglykosidrest ist, in dem ein oder mehrere Wasserstoffatome der OH-Gruppen durch Acetyl oder durch Alkylreste ersetzt sind, dann sind vorzugsweise alle OH-Gruppen, für die der Ersatz möglich ist, durch Acetyl oder durch Alkyl ersetzt.

Unter den in den Verbindungen der Formel IA genannten Alkoxyresten mit 1 bis 8 C-Atomen ist die Methoxygruppe bevorzugt. Unter den in den Verbindungen der Formel IA genannten Alkylresten mit 1 bis 8 C-Atomen ist die Methylgruppe bevorzugt.

Die in den Verbindungen der Formel IA genannten Mono- und Diglykosidreste sind vorzugsweise aus Glucoseeinheiten aufgebaut.

Im folgenden werden bevorzugte Verbindungen IA1 bis IA13 ausgewählt aus den Verbindungen der Formel IA angegeben:

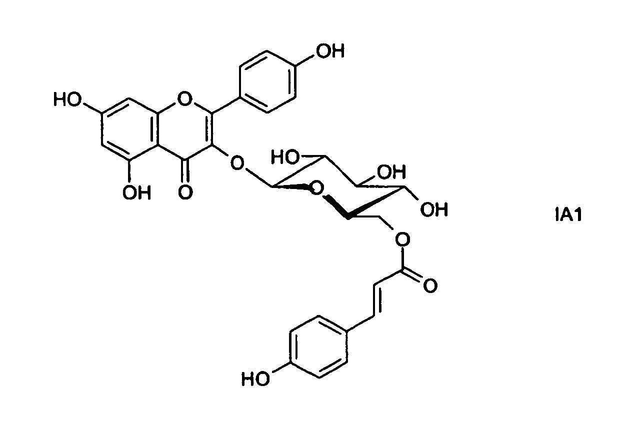

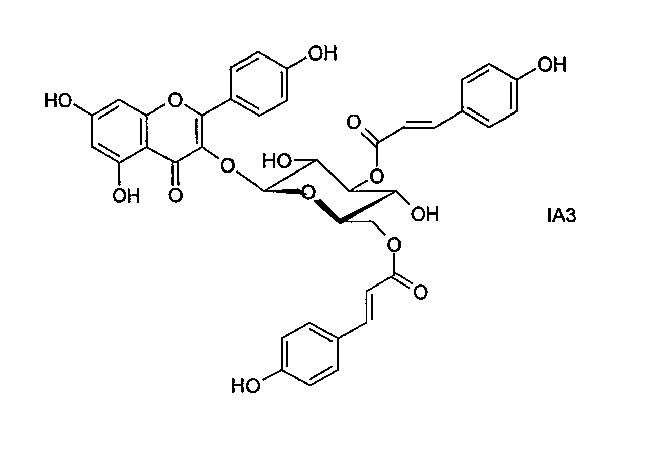

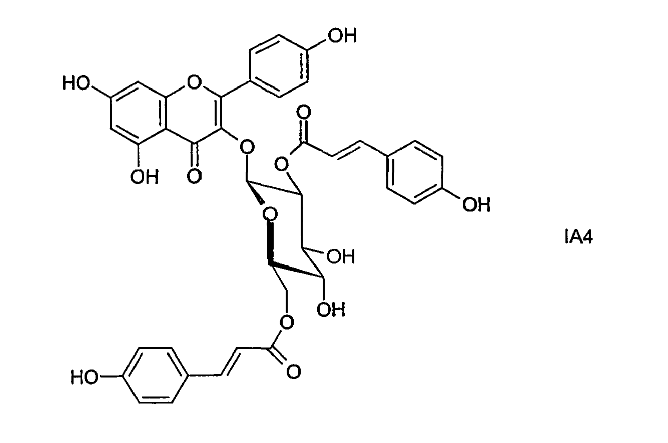

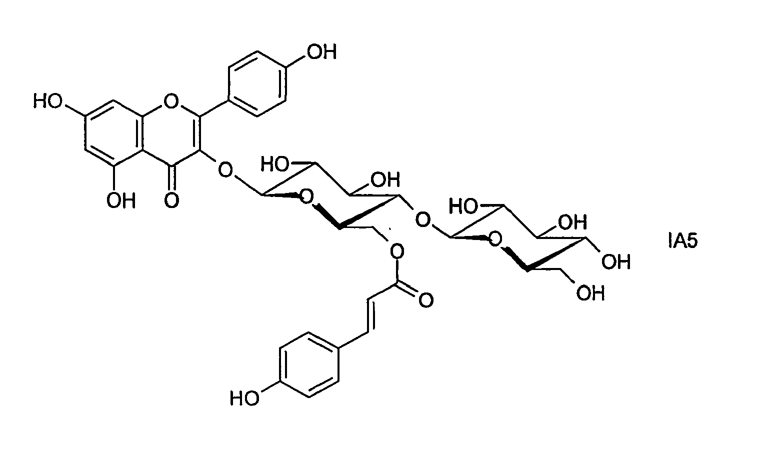

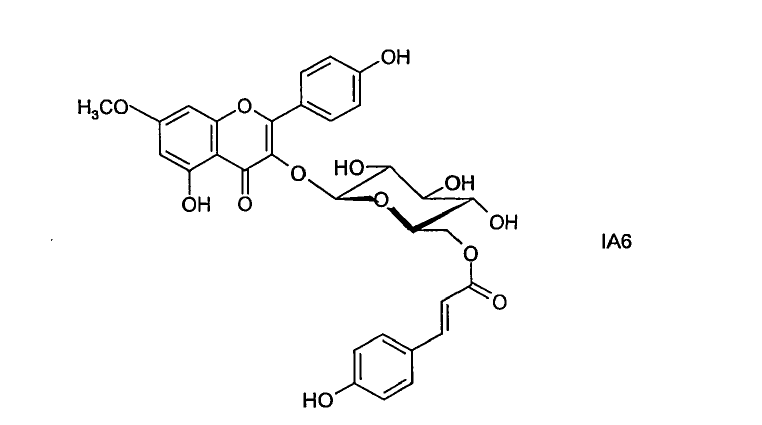

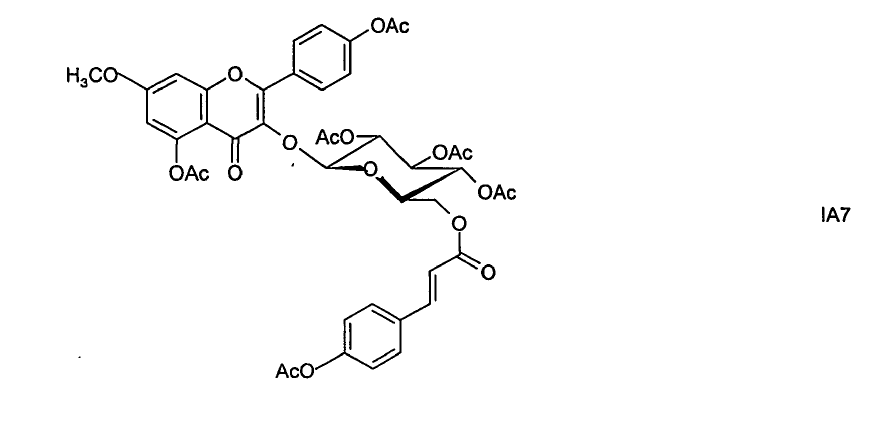

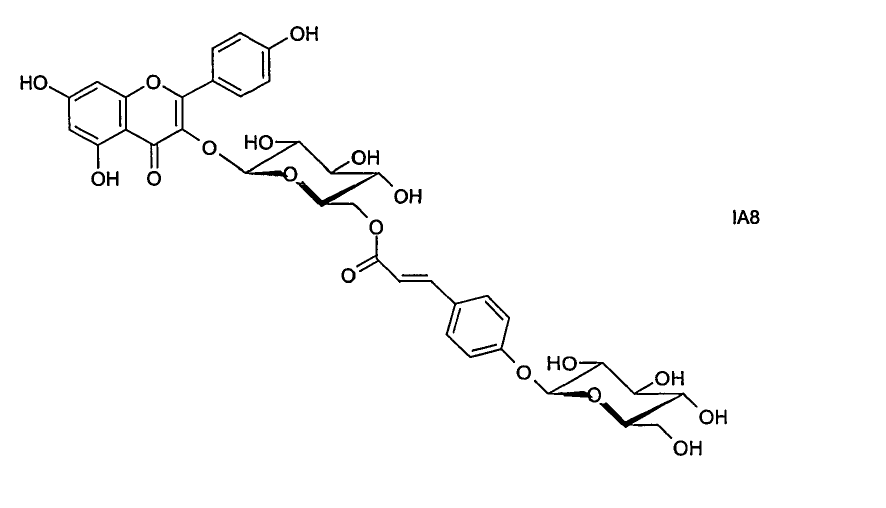

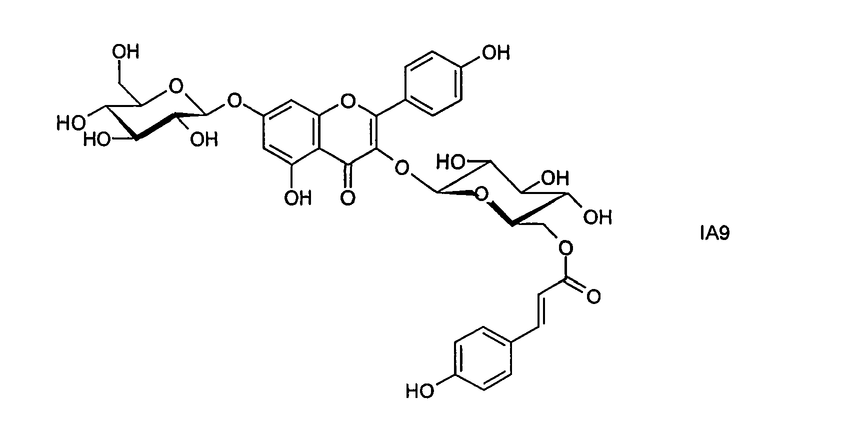

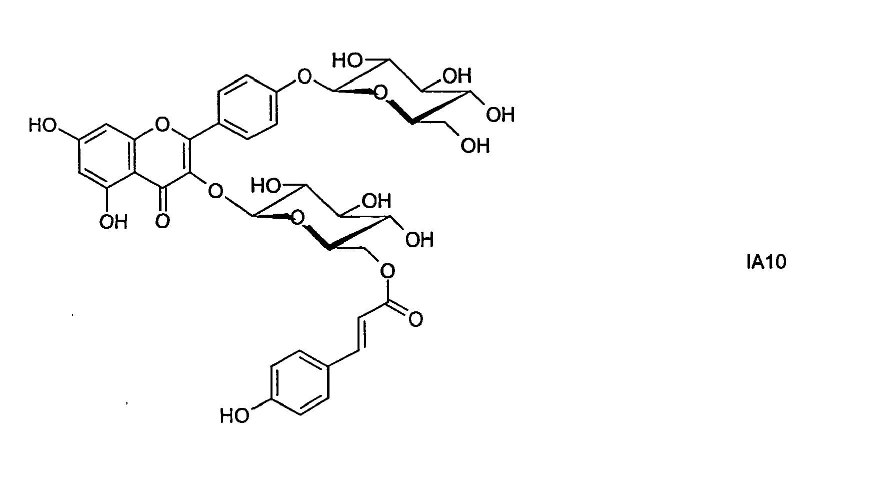

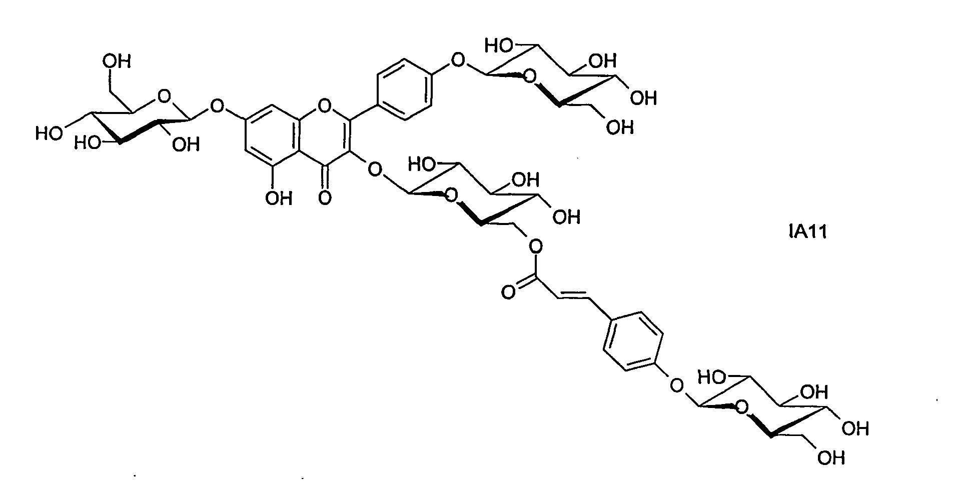

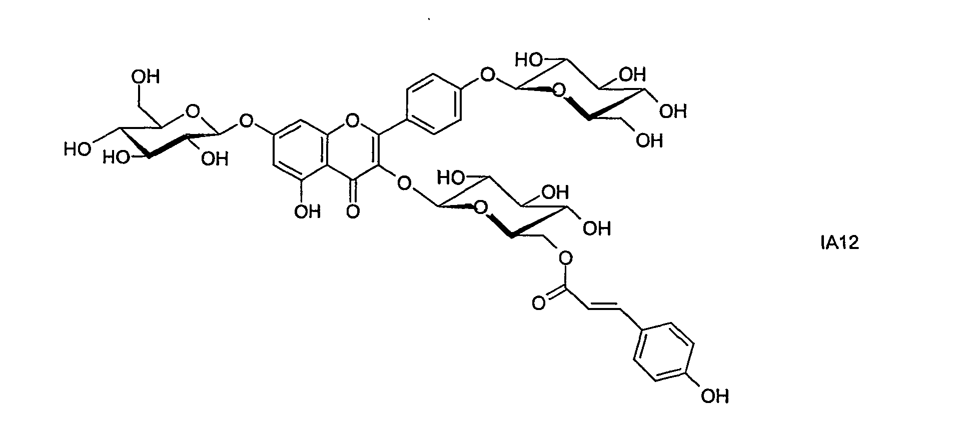

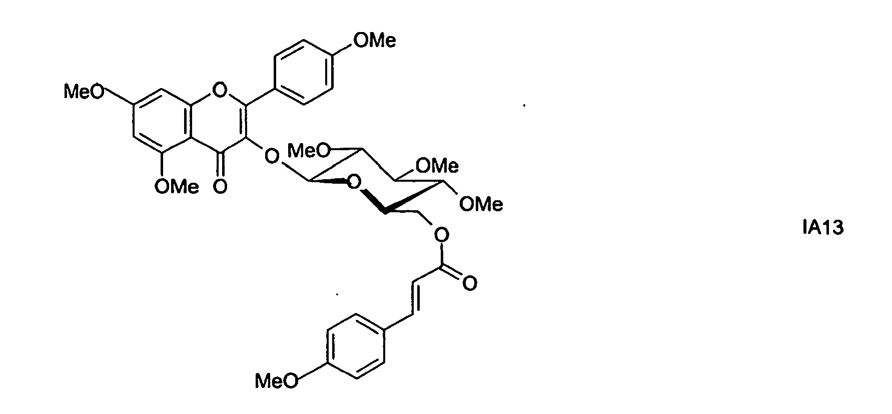

In den oben aufgeführten Verbindungen der Formeln IA1 bis IA13 bedeuten Me Methyl und Ac Acetyl.

Unter den Verbindungen der Formel IA ist insbesondere die Verbindung der Formel IA1 bevorzugt.

In einer weiteren bevorzugten Ausführungsform sind die in den erfindungsgemässen Formulierungen enthaltenen Verbindungen der Formel IA ausgewählt aus den Verbindungen, worin
- R¹, R² und R³: jeweils unabhängig voneinander OH, einen Alkoxyrest mit 1 bis 8 C-Atomen oder einen Monoglykosidrest bedeuten,
- R⁴: ein Mono- oder Diglykosidrest ist, wobei an den Glykosidrest jeweils über eine Gruppe -O- 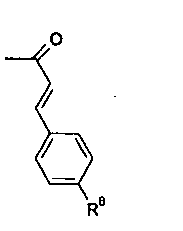 gebunden ist,
- R⁸: OH, einen Alkoxyrest mit 1 bis 8 C-Atomen oder einen Monoglykosidrest bedeutet, und
worin ein oder mehrere Wasserstoffatome in den OH-Gruppen des oder der Glykosidreste jeweils unabhängig voneinander auch durch Alkylreste mit 1 bis 8 C-Atomen ersetzt sein können und wobei an ein oder mehrere Hydroxygruppen der Verbindungen der Formel IA jeweils unabhängig voneinander auch Sulfat oder Phosphat gebunden sein kann.

In diesen Verbindungen der Formel IA bedeuten R¹ bis R³ vorzugsweise jeweils unabhängig voneinander OH oder einen Alkoxyrest mit 1 bis 8 C-Atomen.

Einige Verbindungen der Formel I wie z.B. Tilirosid können aus Pflanzen gewonnen werden, z.B. aus den Pflanzen der Gattung Althaea, Aristolochia, Helianthemum, Lindera, Magnolia, Platanus, Potentilla, Quercus, Rosa, Sida, Sorbus und/oder Tilia. Diese Verbindungen können entweder in isolierter Form oder auch in nicht isolierter Form weiterverarbeitet werden, also z.B. in Form eines Extrakts oder in Form eines aufgereinigten Extrakts oder auch in Form der aus dem Pflanzenextrakt hergestellten Reinsubstanz in kosmetische Formulierungen eingearbeitet werden. Unter den genannten Gattungen sind folgende Arten bevorzugt: Althaea officinalis, Althaea rosea, Aristolochia heterophylla, Helianthemum glomeratum, Lindera megaphylla, Magnolia salicifolia, Platanus acerifolia, Platanus occidentalis, Potentilla anserina, Quercus pubescens, Quercus suber, Quercus laurifolia, Quercus ilex, Quercus imbricaria, Quercus virginiana, Rosa pomifera, Sida rhombifolia, Sida poeppigiana, Sida cordifolia, Sida glaziovii, Sorbus pendula, Tilia argenta und Tilia cordata.

Wenn die erfindungsgemäße kosmetische Formulierung Tilirosid enthält, ist diese Verbindung in einer weiteren bevorzugten Ausführungsform in der Form eines Pflanzenextrakts, eines aufgereinigten Pflanzenextrakts oder in Form der aus dem Pflanzenextrakt hergestellten Reinsubstanz zur Herstellung der kosmetischen Formulierung verwendet worden. In derartigen kosmetischen Formulierungen enthält der Pflanzenextrakt beispielsweise 1 bis 100 Gew.% Tilirosid. In einer Ausführungsform enthält der Pflanzenextrakt vorzugsweise 5 bis 90 Gew.% Tilirosid. In einer weiteren Ausführungsform enthält der Pflanzenextrakt vorzugsweise 30 bis 100 Gew.%, besonders bevorzugt 60 bis 100 Gew.% und insbesondere bevorzugt 90 bis 100 Gew.% Tilirosid. In einer weiteren bevorzugten Ausführungsform ist der Pflanzenextrakt durch Extraktion der Pflanze Sida glaziovii gewonnen worden.

Bei allen erfindungsgemäßen Verwendungen, in denen Tilirosid zum Einsatz kommt, z.B. wenn Tilirosid als UV-Filter, als Radikalfänger und/oder Antioxidans, gegen oxidativen Stress, zur Vermeidung der Hautalterung oder als Wirkstoff mit anti-allergischer, anti-inflammatorischer, entzündungshemmender oder anti-irritativer Wirkung in kosmetischen Formulierungen eingesetzt wird, kann Tilirosid z.B. in Form einer synthetisch gewonnenen Substanz, in Form eines Pflanzenextrakts, eines aufgereinigten Pflanzenextrakts oder als Einzelsubstanz bzw. in Form einer aus dem Pflanzenextrakt gewonnenen Reinsubstanz verwendet werden. In einer bevorzugten Ausführungsform wird Tilirosid hierbei in der Form eines Pflanzenextrakts, eines aufgereinigten Pflanzenextrakts oder in Form der aus dem Pflanzenextrakt hergestellten Reinsubstanz verwendet.

Die Verbindungen der Formel IA können nach Methoden, die dem Fachmann wohl bekannt und in der Literatur beschrieben sind (z.B. in Standard-Werken wie Houben-Weyl, Methoden der organischen Chemie, Georg-Thieme-Verlag, Stuttgart), gewonnen oder hergestellt werden.

Beispielsweise kommt Tilirosid in Pflanzen vor und kann durch Extraktion gewonnen werden. Die Herstellung der Pflanzenextrakte erfolgt durch übliche Methoden der Extraktion der Pflanzen bzw. Pflanzenteile. Geeignete Extraktionsverfahren können sein: Mazeration, Remazeration, Digestion, Bewegungsmazeration, Wirbelextraktion, Ultraschallextraktion, Gegenstromextraktion, Perkolation, Reperkolation, Evakolation, Diakolation oder Festflüssig-Extraktion unter kontinuierlichem Rückfluss, die in einem Soxhlet-Extraktor durchgeführt wird.

Als Lösungsmittel für die Extraktion kann beispielsweise Wasser oder ein Alkohol verwendet werden.

Es ist dem allgemeinen Wissen des Fachmanns zuzurechnen, wie diese Extraktionen im Einzelnen durchgeführt werden und die erhaltenen Rohextrakte durch allgemein geläufige Methoden aufgereinigt werden können.

Ein möglicher Syntheseweg für Tilirosid ist z.B. auch in B. Vermes, H. Wagner, Stud. Org. Chem. (Amsterdam) (1982), Volume date 1981, 11 (Flavonoids, Bioflavonoids), 161-167 und in B. Vermes, V.M. Chari, H. Wagner, Helv. Chim. Acta (1981), 64(4), 1964-1967 beschrieben.

Die Synthese von Tilirosid ist in Schema 1 wiedergegeben.

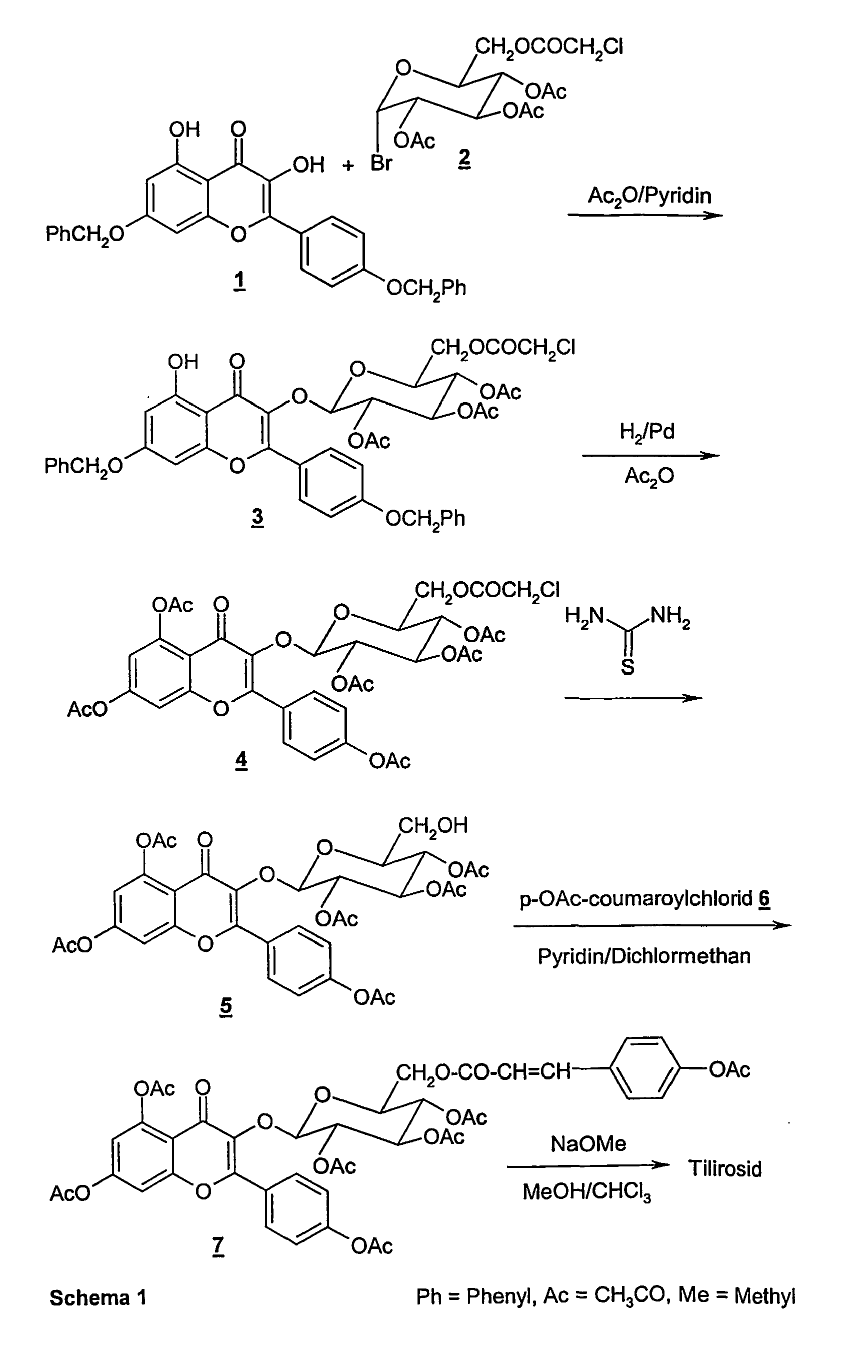

4',7-Dibenzylkaempferol (**1**) [H. Wagner, H. Danninger, O. Seligmann, M. Nógrádi, L. Farkas, N. Farnsworth, Chem. Ber. 103 (1978) 3768] wird in Gegenwart von Ag₂CO₃ und Pyridin mit 2,3,4-Tri-O-acetyl-6-O-chloroacetyl-β-D-glucopyranosylbromid (**2**) zu Verbindung **3** umgesetzt. Die Verbindung **2** kann nach der in D.Y. Gagniere, P.J.A. Wottero, Carbohydrate Res. 28 (1973) 1965 beschriebenen Methode hergestellt werden. Die katalytische Debenzylierung und anschließende vorsichtige Acetylierung der Verbindung **3** liefert Verbindung **4** aus der nach Entfernung der Chloracetyl-Gruppe mit Thioharnstoff Verbindung **5** erhalten werden kann. In dieser Verbindung ist lediglich eine Hydroxylgruppe frei, sodaß die Veresterung der Verbindung **5** selektiv verlaufen kann. Die Veresterung mit dem Säurechlorid p-Acetylcoumaroylchlorid **6** kann in einer Mischung aus Pyridin und Dichloromethan durchgeführt werden. Damit die Veresterung vollständig abläuft ist ein Überschuß an Säurechlorid und eine lange Reaktionszeit (ca. 96h) bei Raumtemperatur notwendig. Der letzte Schritt, die selektive Verseifung der 7 Acetylgruppen in Verbindung **7**, kann nach der in G. Zemplén, Chem. Ber. 59 (1926) 1258 beschriebenen Methode durchgeführt werden. Hierbei wird unter Verwendung einer katalytischen Menge an NaOCH₃ und einer kalkulierten Menge an Methanol gearbeitet.

Andere Verbindungen der Formel IA können durch routinemäßige Abwandlung der in Schema 1 gezeigten Synthese erhalten werden. Hierbei werden je nach Zielmolekül andere Edukte, d.h. andere gegebenenfalls geschützte Flavonoide, Zuckerkomponenten und Reste, die an die Zuckerkomponente angehängt werden sollen, verwendet.

Die Veresterung glykosidischer OH-Gruppen mit aromatischen Sulfonsäure-Einheiten kann beispielsweise nach der in A.B. Foster et al., J. Chem. Soc. (1954) 3625-3629 beschriebenen Methode erfolgen. Hiernach kann die Zuckerkomponente z.B. mit einem entsprechenden aromatischen Sulfonsäurechlorid in Pyridin zur Reaktion gebracht werden.

Die Veretherung glykosidischer OH-Gruppen mit aromatischen Resten kann beispielsweise nach der in P. Beraud et al., Tetrahedron Let. 30(3) (1989) 325-326 beschriebenen Methode erfolgen. Bei dieser Mitsunobu-Reaktion findet die Veretherung beispielsweise derart statt, dass die Zuckerkomponente zusammen mit Triphenylphosphin PPh₃ in Pyridin gelöst und mit einer entsprecheneden Phenolkomponente und Diethylazodicarboxylat zur Reaktion gebracht wird.

Die Veretherung glykosidischer OH-Gruppen mit Resten gesättigter Kohlenwasserstoffe kann beispielsweise nach der in M. Goebel et al., Tetrahedron 53(9) (1997) 3123-3134 beschriebenen Methode erfolgen. Die Veretherung findet z.B. derart statt, dass die Zuckerkomponente in trockenem Dimethylformamid unter Inertgas vorsichtig mit Natriumhydrid versetzt und danach mit einem geeigneten Alkylierungsreagenz wie z.B. einem entsprechenden Bromid vorsichtig umgesetzt wird.

Der Anteil der Verbindungen der Formel IA in der kosmetischen Formulierung beträgt vorzugsweise von 0,001 bis 20 Gew.%, besonders bevorzugt von 0,01 bis 10 Gew.% und insbesondere bevorzugt von 0,05 bis 5 Gew.% bezogen auf die gesamte kosmetische Formulierung. Ganz außerordentlich bevorzugt beträgt der Anteil der Verbindungen der Formel I in der kosmetischen Formulierung von 0,05 bis 2 Gew.% bezogen auf die gesamte kosmetische Formulierung.

Die schützende Wirkung der erfindungsgemäßen kosmetischen Formulierungen gegen UV-Strahlung kann verbessert werden, wenn die Formulierung neben den Verbindungen der Formel IA einen oder mehrere weitere UV-Filter enthält.

Prinzipiell kommen alle UV-Filter für eine Kombination in Frage. Besonders bevorzugt sind solche UV-Filter, deren physiologische Unbedenklichkeit bereits nachgewiesen ist. Sowohl für UV-A- als auch UVB-Filter gibt es viele aus der Fachliteratur bekannte und bewährte Substanzen, z.B.

Benzylidenkampferderivate wie
- 3-(4'-Methylbenzyliden)-dl-kampfer (z.B. Eusolex^{®} 6300),
- 3-Benzylidenkampfer (z.B. Mexoryl^{®} SD),
- Polymere von N-{(2 und 4)-[(2-oxoborn-3-yliden)methy)]benzy}acrylamid (z.B. Mexoryl^{®} SW),
- N,N,N-Trimethyl-4-(2-oxobom-3-ylidenmethyl)anilinium methylsulfat (z.B. Mexoryl^{®} SK) oder
- α-(2-Oxoborn-3-yliden)toluol-4-sulfonsäure (z.B. Mexoryl^{®} SL),

Benzoyl- oder Dibenzoylmethane wie
- 1-(4-tert-Butylphenyl)-3-(4-methoxyphenyl)propan-1,3-dion (z.B. Eusolex^{®} 9020) oder
- 4-Isopropyldibenzoylmethan (z.B. Eusolex^{®} 8020),

Benzophenone wie
- 2-Hydroxy-4-methoxybenzophenon (z.B. Eusolex^{®} 4360) oder
- 2-Hydroxy-4-methoxybenzophenon-5-sulfonsäure und ihr Natriumsalz (z.B. Uvinul^{®} MS-40),

Methoxyzimtsäureester wie
- Methoxyzimtsäureoctylester (z.B. Eusolex^{®} 2292),
- 4-Methoxyzimtsäureisopentylester, z.B. als Gemisch der Isomere (z.B. Neo Heliopan^{®} E 1000),

Salicylatderivate wie
- 2-Ethylhexylsalicylat (z.B. Eusolex^{®} OS),
- 4-lsopropylbenzylsalicylat (z.B. Megasol^{®}) oder
- 3,3,5-Trimethylcyclohexylsalicylat (z.B. Eusolex^{®} HMS),

4-Aminobenzoesäure und Derivate wie
- 4-Aminobenzoesäure,
- 4-(Dimethylamino)benzoesäure-2-ethylhexylester (z.B. Eusolex^{®} 6007),
- ethoxylierter 4-Aminobenzoesäureethylester (z.B. Uvinul^{®} P25),

Benzimidazolderivate wie
- 2-Phenylbenzimidazol-5-sulfonsäure sowie ihre Kalium-, Natrium- und Triethanolaminsalze (z.B. Eusolex^{®} 232),
- 2,2'-(1,4-Phenylen)bis-(1H-benzimidazol-4,6-disulfonsäure, Mononatriumsalz) (CAS-Nr. 180 898-37-7),
- 2,2'-(1,4-Phenylen)bis-(1H-benzimidazol-5-sulfonsäure) sowie ihre Kalium-, Natrium- und Triethanolaminsalze,
und weitere Substanzen wie
- 2-Cyano-3,3-diphenylacrylsäure-2-ethylhexylester (z.B. Eusolex^{®} OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl^{®} SX),
- 2,4,6-Trianilino-(p-carbo-2'-ethylhexyl-1'-oxi)-1,3,5-triazin (z.B. Uvinul^{®} T 150),
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),
- α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1),
- 2,2'-Methylen-bis-(6-(2H-benzotriazol-2-yl)-4-(1,1,3,3-tetramethylbutyl)phenol) (CAS-Nr. 103 597-45-1),
- 2,4-bis-{[4-(2-Ethyl-hexytoxy)2-hydroxyl]-phenyl}-6-(4-methoxyphenyl)-1,3,5-triazin (CAS-Nr. 103 597-45-, 187 393-00-6).

Die in der Liste aufgeführten Verbindungen sind nur als Beispiele aufzufassen. Selbstverständlich können auch andere UV-Filter verwendet werden. Diese organischen UV-Filter werden wie auch die Verbindungen der Formel IA in der Regel in einer Menge von 0,5 bis 20 Gew.-%, vorzugsweise in einer Menge von 1 bis 15 Gew.-% und insbesondere bevorzugt in Mengen von 2 bis 8 Gew.-% je Einzelsubstanz in kosmetische Formulierungen eingearbeitet. Insgesamt enthalten die kosmetischen Zubereitungen üblicherweise bis zu 40 Gew.-%, vorzugsweise 5 bis 25 Gew.-% solcher organischer UV-Filter.

Als anorganische UV-Filter sind solche aus der Gruppe der Titandioxide wie z.B. gecoatetes Titandioxid (z.B. Eusolex^{®} T-2000, Eusolex^{®} T-AQUA), Zinkoxide (z.B. Sachtotec^{®}), Eisenoxide oder auch Ceroxide denkbar. Diese anorganischen UV-Filter werden in der Regel in einer Menge von 0,5 bis 20 Gew.-%, vorzugsweise 2 bis 10 Gew.-%, in kosmetische Formulierungen eingearbeitet.

Werden verschiedene anorganische oder organische UV-Filter eingesetzt, so können diese in nahezu beliebigen Verhältnissen zueinander verwendet werden. Üblicherweise liegen die Verhältnisse der einzelnen Substanzen zueinander im Bereich 1:10 - 10:1, vorzugsweise im Bereich 1:5 - 5:1 und insbesondere bevorzugt im Bereich 1:2 - 2:1. Werden UV-A- neben UV-B-Filtern eingesetzt, so ist es für die meisten Anwendungen von Vorteil, wenn der Anteil an UV-B-Filtern überwiegt und das Verhältnis von UV-A-Filtern : UV-B-Filtern im Bereich 1:1 bis 1:10 liegt.

Neben den Verbindungen der Formel IA sind als bevorzugte Verbindungen mit UV-filtemden Eigenschaften für die kosmetischen Zubereitungen 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester und gecoatetes Titandioxid zu nennen.

Die schützende Wirkung gegen oxidativen Stress bzw. gegen die Einwirkung von Radikalen kann weiter verbessert werden, wenn die Formulierung ein oder mehrere weitere Antioxidantien enthält.

Es gibt viele aus der Fachliteratur bekannte und bewährte Substanzen, die verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydrollponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Formulierungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004),

Der Anteil des einen oder der mehreren Antioxidantien in der kosmetischen Formulierung beträgt vorzugsweise von 0,001 bis 5 Gew.%, besonders bevorzugt von 0,01 bis 2 Gew.% bezogen auf die gesamte Formulierung.

Die schützende Wirkung der erfindungsgemäßen kosmetischen Formulierungen gegen UV-Strahlung und/oder oxidativen Stress kann auch verbessert werden, wenn die Formulierung neben den Verbindungen der Formel IA ein oder mehrere Verbindungen ausgewählt aus Flavonoiden und Coumaranonen enthält. Als Flavonoide werden die Glykoside von Flavanonen, Flavonen, 3-Hydroxyflavonen (= Flavonolen), Auronen, Isoflavonen und Rotenoiden aufgefaßt [Römpp Chemie Lexikon, Band 9, 1993]. Im Rahmen der vorliegenden Erfindung werden hierunter jedoch auch die Aglykone, d.h. die zuckerfreien Bestandteile, und die Derivate der Flavonoide und der Aglykone verstanden. Weiterhin wird im Rahmen der vorliegenden Erfindung unter dem Begriff Flavonoid auch Anthocyanidin (Cyanidin) verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und lsoflavonen, insbesondere von Flavanonen, Flavonen, 3-Hydroxyflavonen und Auronen, ab.

Die Flavonoide sind vorzugsweise ausgewählt aus folgenden Verbindungen: 4,6,3',4'-Tetrahydroxyauron, Quercetin, Rutin, Isoquercetin, Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin) sowie deren Sulfaten und Phosphaten. Unter den Flavonoiden sind insbesondere Rutin und Troxerutin bevorzugt. Ganz außerordentlich bevorzugt ist Troxerutin.

Unter den Coumaranonen ist 4,6,3',4'-Tetrahydroxybenzylcoumaranon-3 bevorzugt.

Der Anteil der einen oder mehreren Verbindungen ausgewählt aus Flavonoiden und Coumaranonen in der kosmetischen Formulierung beträgt vorzugsweise von 0,001 bis 5 Gew.%, besonders bevorzugt von 0,01 bis 2 Gew.% bezogen auf die gesamte Formulierung.

Die erfindungsgemäßen Formulierungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Formulierungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die erfindungsgemäßen Formulierungen können weiter als Inhaltsstoff auch Ectoin [(S)-1,4,5,6-Tetrahydro-2-methyl-4-pyrimidincarbonsäure] enthalten und bewirken dann einen Schutz von Zellen der Haut, insbesondere einen Schutz der Langerhanszellen. Besonders vorteilhaft sind kosmetische Formulierungen enthaltend Tilirosid und Ectoin.

Durch die Zugabe von 1-(2-Hydroxyaryl)-alkan-1-on-oximen (wie z.B. in der EP 0 149 242 beschrieben) und vorzugsweise von 2-Hydroxy-5-methyl-laurophenonoxim erhält die erfindungsgemäße Formulierung eine vorteilhafte anti-inflammatorische Wirkung. Besonders vorteilhaft sind kosmetische Formulierungen enthaltend Tilirosid und 2-Hydroxy-5-methyl-laurophenonoxim, worin die genannten Substanzen in einem Gewichtsverhältnis von 1:10 bis 10:1 enthalten sind. Anwendungsformen derartiger Formulierungen sind z.B. After-Sun-Präparate.

Weiterhin sind auch erfindungsgemäße Formulierungen bevorzugt, die Tilirosid und 4,6,3',4'-Tetrahydroxybenzylcoumaranon-3 enthalten. In diesen Formulierungen sind die genannten Substanzen in einem Gewichtsverhältnis von 1 : 10 bis 10 : 1 enthalten.

In die erfindungsgemäßen Formulierungen können auch weitere Wirkstoffe eingearbeitet werden, z.B.
- Hydroxyectoin [(S,S)-1,4,5,6-Tetrahydro-5-hydroxy-2-methyl-4-pyrimidincarbonsäure]
- Wirkstoffe, die zur Wundbehandlung dienen können, wie z.B. Allantoin
- Insekt-Repellentien wie z.B. 3-[N-n-butyl-N-acetyl]-aminopropionsäure-ethylester [CAS-Nr. 52304-36-6]
- Sorbit für die Hautpflege [z.B. Karion^{®}F flüssig oder Karion^{®}FP flüssig]
- Biotin
- anti-ageing-Produkte wie z.B. Mischungen enthaltend Hydroxyprolin oder Derivate von Hydroxyprolin, z.B. Mischungen enthaltend Lecithin, Hydroxyprolindipalmitat, Sitosterol, Linolsäure, Tocopherol, Natriumascorbat, Mannit, Phenoxyethanol, Methylparaben, Ethylparaben, Propylparaben, Butylparaben, Wasser [z.B. RonaCare^{™} ASC III^{®}] oder z.B. Mischungen enthaltend Lecithin, hydroxyliertes Lecithin, L-Hydroxyprolin, Dinatrium Rutinyldisulfat, Phenoxyethanol, Mannit, Magnesiumascorbylphosphat, Methylparaben, Ethylparaben, Propylparaben, Butylparaben, Sitosterol, Tocopherol, Natriumascorbat, Wasser [z.B. RonaCare^{™} VTA]
- Bisabolol.

Die Verbindungen der Formel IA können in der üblichen Weise in kosmetische Formulierungen eingearbeitet werden. Geeignet sind Formulierungen für eine äußerliche Anwendung, beispielsweise als Creme, Lotion, Gel, oder als Lösung, die auf die Haut aufgesprüht werden kann. Dabei ist es bevorzugt, wenn die Zubereitung mindestens eine Öl- und mindestens eine Wasser-Phase enthält.

Als Anwendungsform der erfindungsgemäßen kosmetischen Formulierungen seien z.B. genannt: Lösungen, Emulsionen, PIT-Emulsionen, Suspensionen, Pasten, Salben, Gele, Cremes, Seifen, tensidhaltige Reinigungspräparate, Lotionen, Öle, Puder, Sprays und Aerosole. Weitere Anwendungsformen sind z.B. Sticks, Shampoos und Duschbäder. Zusätzlich zu den Verbindungen der Formel IA können der Formulierung beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer, Filmbildner, Verdickungsmittel, Feuchthaltemittel.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Die Emulsionen können in verschiedenen Formen vorliegen. So können sie z.B. eine Emulsion oder Mikroemulsion vom Typ Wasser-in-Öl (W/O), oder vom Typ Öl-in-Wasser (O/W), oder eine multiple Emulsion, beispielsweise vom Typ Wasser-in-Öl-in-Wasser (W/O/W), darstellen.

Die kosmetischen Formulierungen können auch als emulgatorfreie, disperse Zubereitungen, vorliegen. Sie können beispielsweise Hydrodispersionen oder Pikkering-Emulsionen darstellen.

Die kosmetischen Formulierungen können auch als PIT-Emulsionen oder als Hydrogele vorliegen. Die kosmetischen Formulierungen können auch Liposomen, die beispielsweise Wirkstoffe umschliessen, enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Pasten, Salben, Gele und Cremes können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, lsothionate, lmidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Alle Verbindungen oder Komponenten, die in den kosmetischen Formulierungen verwendet werden können, sind entweder bekannt und käuflich erwerbbar oder können nach bekannten Verfahren synthetisiert werden.

Die erfindungsgemäße kosmetische Zubereitung eignet sich besonders zum Schutz menschlicher Haut vor den schädigenden Einflüssen der UV-Anteile im Sonnenlicht, daneben bieten sie auch Schutz gegen Alterungsprozesse der Haut sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrig-alkoholischer Gele bzw. Lösungen, als feste Stifte vorliegen oder als Aerosol konfektioniert sein.

Die Formulierung kann kosmetische Adjuvanzien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer einer oder mehrerer Verbindungen der Formel IA und gegebenenfalls noch weiteren Lichtschutzfiltern Fettalkohole, Fettsäuren, Fettsäureester, insbesondere Triglyceride von Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Die erfindungsgemäße kosmetische Zubereitung kann auch als alkoholisches Gel vorliegen, welches einen oder mehrere Niedrigalkohole oder -polyole, wie Ethanol, Propylenglycol oder Glycerin, und ein Verdickungsmittel wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Formulierung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Formulierung vor oder nach dem Shamponieren, vor oder nach dem Färben oder Entfärben bzw. vor oder nach der Dauerwelle aufgetragen wird. Es kann auch eine Formulierung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die kosmetische Formulierung kann außer der oder den Verbindungen der Formel IA und weiteren UV-Filtern verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäßen kosmetischen Zubereitungen können mit Hilfe von Techniken hergestellt werden, die dem Fachmann wohl bekannt sind.

Zum Schutz der Haut und/oder natürlicher oder sensibilisierter Haare vor Sonnenstrahlen wird auf die Haut oder die Haare eine kosmetische Zubereitung, enthaltend eine oder mehrere Verbindungen der Formel IA aufgetragen. Als sensibilisierte Haare werden dabei Haare verstanden, welche einer chemischen Behandlung, wie einer Dauerwellenbehandlung, einem Färbe- oder Entfärbeprozeß unterzogen worden sind.

Ferner wirken die Verbindungen der Formel IA auch stabilisierend auf die Formulierung. Bei der Verwendung in entsprechenden Produkten bleiben diese daher auch länger stabil und verändern ihr Aussehen nicht. Insbesondere bleibt auch bei längerdauernder Anwendung bzw. längerer Lagerung die Wirksamkeit der Inhaltsstoffe, z.B. Vitamine, erhalten. Dies ist besonders vorteilhaft bei der Zusammensetzungen zum Schutz der Haut gegen die Einwirkung von UV-Strahlen, da diese Kosmetika besonders hohen Belastungen durch die UV-Strahlung ausgesetzt sind.

Ein weiterer Gegenstand der Erfindung betrifft die Stabilisierung von UV-Filtem. Eine bekannte und leistungsfähige Klasse von Lichtschutzfiltersubstanzen wird von den Dibenzoylmethanderivaten gebildet. Nachteilig ist jedoch, dass diese Substanzen sehr leicht durch UV-Licht zersetzt werden und damit ihre schützenden Eigenschaften verlorengehen. Als Beispiel für einen kommerziell erhältlichen Lichtschutzfilter aus dieser Verbindungsklasse sei das 4-(tert.-Butyl)-4'-methoxydibenzoylmethan angeführt, welches die unten gezeigte Struktur aufweist.

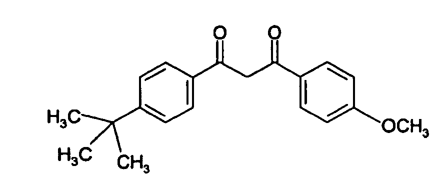

Überraschend hat sich nun gezeigt, dass Verbindungen der Formel IA eine sehr gute Stabilisierungswirkung für die Dibenzoylmethane, insbesondere 4-(tert.-Butyl)-4-methoxydibenzoylmethan, aufweisen. Eine besonders hohe Stabilisierungswirkung wurde für Tilirosid gefunden. Hierbei kann Tilirosid als Reinsubstanz oder in Form eines Pflanzenextrakts zur Stabilisierung verwendet werden. In einer bevorzugten Ausführungsform wird Tilirosid in der Form eines Pflanzenextrakts, eines aufgereinigten Pflanzenextrakts oder in Form der aus dem Pflanzenextrakt hergestellten Reinsubstanz verwendet. Somit ist es nun möglich Lichtschutzmittel unter Verwendung von Dibenzoylmethanen herzustellen, die auch bei längerer Sonneneinwirkung, beispielsweise während eines mehrstündigen Sonnenbades, keine oder nur eine geringe Verringerung der Schutzwirkung gegen UV-Strahlen aufweisen.

Weiterhin Gegenstand der vorliegenden Erfindung ist sulfatisiertes Tilirosid, also Tilirosid, das dadurch gekennzeichnet ist, dass an eine oder an mehrere Hydroxygruppen Sulfat gebunden ist.

Auch ohne weitere Ausführungen wird davon ausgegangen, dass ein Fachmann die obige Beschreibung in weitestem Umfang nutzen kann. Die bevorzugten Ausführungsformen sind deswegen lediglich als beschreibende, keinesfalls als in irgendeiner Weise limitierende Offenbarung aufzufassen.

Die vollständige Offenbarung aller vor- und nachstehend aufgeführten Anmeldungen und Veröffentlichungen sind durch Bezugnahme in diese Anmeldung eingeführt.

Die folgenden Beispiele sollen die vorliegende Erfindung verdeutlichen. Sie sind jedoch keinesfalls als limitierend zu betrachten.

Alle Verbindungen oder Komponenten, die in den kosmetischen Formulierungen verwendet werden können, sind entweder bekannt und käuflich erhältlich oder können nach bekannten Methoden synthetisiert werden.

Die INCI-Namen der verwendeten Rohstoffe sind wie folgt (die INCI-Namen werden definitionsgemäß in Englischer Sprache angegeben):

| **Rohstoff** | **INCI-Name** |
|---|---|
| Abil WE 09 | Polyglyceryl-4-lsostearate, Cetyl |
| | Dimethicone Copolyol, Hexyl Laurate |
| Antaron V-220 | PVP/Eicosene Copolymer |
| Arlacel 80 | Sorbitan Oleate |
| Arlacel 165 V | Glyceryl Stearate, PEG-100 Stearate |
| Avocadoöl | Persea Gratissima |
| Bienenwachs | Beeswax |
| Biobase^{™} EP | Glyceryl Stearate, Cetearyl Alcohol, |
| | Sodium Stearoyl Lactylate, Lecithin |
| Carbopol ETD 2050 | Carbomer |
| Cetiol V | Decyl Oleate |
| Cetylalkohol | Cetyl Alcohol |
| Cetylisononanoat | Cetyl Isononanoate |
| Cutina HR | Hydrogenated Castor Oil |
| Dimeticon | Dimethicone |
| Eusolex^{®}232 | Phenylbenzimidazole Sulfonic Acid |
| Eusolex^{®} 2292 | Octyl Methoxycinnamate, BHT |
| Eusolex^{®} 6300 | 4-Methylbenzylidene Camphor |
| Eusolex 8300 | 4-Methylbenzylidene |
| Eusolex^{®} 9020 | Butyl Methoxydibenzoylmethane |
| Eusolex^{®}HMS | Homosalate |
| Eusolex T-Aqua | Aqua (Water), Titanium Dioxide, |
| | Alumina, Sodium Metaphosphate, |
| | Phenoxyethanol, Sodium Methyl- |
| | paraben |
| Eutanol G | Octyldodecanol |
| Germaben ll | Propylene Glycol, Diazolidinyl Urea, |
| | Methylparaben, Propylparaben |
| Germaben II-E | Propylene Glycol, Diazolidinyl Urea, |
| | Methylparaben, Propylparaben |
| Glycerin | Glycerin |
| Glycerin (87%) | Glycerin |
| Glycerin (87% reinst) | Glycerin |
| Glycerin, wasserfrei | Glycerin |
| Hetester PHA | Propylene Glycol Isoceteth-3 Acetate |
| Hexyllaurat | Hexyl Laurate |
| Imwitor 960 K Schuppen | Glyceryl Stearate SE |
| Isolan PDI | Diisostearoyl Polyglyceryl-3-Diisostearat |
| Isopropylmyristat | Isopropyl Myristate |
| Isopropylpalmitat | Isopropyl Palmitate |
| Jojobaöl | Buxus Chinensis (Jojoba Oil) |
| Karion F flüssig | Sorbitol |
| Keltrol RD | Xanthan Gum |
| Magnesiumsulfat | Magnesium Sulfate |
| Magnesiumsulfat-Heptahydrat | Magnesium Sulfate |
| Methyl-4-hydroxybenzoat | Methylparaben |
| Miglyol 812 | Caprylic/Capric Triglyceride |
| Miglyol 812 N | Caprylic/Capric Triglyceride |
| Miglyol 812, Neutralöl | Caprylic/Capric Triglyceride |
| Mirasil CM5 | Cyclomethicone |
| Mirasil DM 350 | Dimethicone |
| Montanov 68 | Cetearyl Alcohol, Cetearyl Glucoside |
| Natriumchlorid | Sodium Chloride |
| Natronlauge, 10%ig | Sodium Hydroxide |
| Oxynex^{®}K | PEG-8, Tocopherol, Ascorbyl Palmitate, |
| | Ascorbic Acid, Citric Acid |
| Panthenol-D | Panthenol |
| Paracera M | Microwax |
| Paraffinöl, fl. | Mineral Oil |
| Parfümöl TND-2417 | Parfum |
| Pemulen TR-1 | Acrylates/C₁₀₋₃₀ Alkyl Acrylate |
| | Crosspolymer |
| Pemulen^{®} TR-2 | Acrylates/C₁₀₋₃₀ Alkyl Acrylate |
| | Crosspolymer |
| Performa^{®} V 825 | Synthetic Wax |
| Polyglyceryl-2-Dipolyhydroxy- | Polyglyceryl-2 Dipolyhydroxystearate |
| stearat | |
| Prisorine 2021 | Isopropyl Isostearate |
| Propandiol-1,2 | Propylene Glycol |
| Propyl-4-hydroxybenzoat | Propylparaben |
| Rhodicare S | Xanthan Gum |
| RonaCare^{™} ASC III | Aqua, Lecithin, Dipalmitoyl |
| | Hydroxyproline, Phenoxyethanol, Tall |
| | Oil Sterol, Linoleic Acid, Tocopherol, |
| | Sodium Ascorbate, Mannitol, |
| | Methylparaben, Ethylparaben, |
| | Propylparaben, Butylparaben |
| RonaCare^{™} Bisabolol | Bisabolol |
| RonaCare^{™} Ectoin | Ectoin |
| RonaCare^{™} LPO | Lauryl p-Cresol Ketoxime |
| RonaCare^{™} Tocopherolacetat | Tocopheryl Acetate |
| Sepigel 305 | Polyacrylamide, C₁₃₋₁₄ Isoparaffin, |
| | Laureth-7 |
| SFE 839 | Cyclopentasiloxane, Dimethicone/ |
| | Vinyldimethicone Crosspolymer |
| Shea Butter | Shea Butter |
| Steareth-2 | Steareth-2 |
| Steareth-10 | Steareth-10 |
| Stearinsäure | Stearic Acid |
| DL-α-Tocopherolacetat | Tocopherol Acetate |
| Triethanolamin | Triethanolamine |
| Triethanolamin reinst | Triethanolamine |
| Wasser, demineralisiert | Aqua (Water) |
| Zinkstearat | Zinc Stearate |

### Beispiele

### Beispiel A

### Herstellung von sulfatisiertem Tilirosid, Natriumsalz

29,7 g Tilirosid (50 mmol) werden unter Rühren mit 200 ml Wasser und 19,4 g 32%iger Natronlauge (155,2 mmol) versetzt. Anschließend wird 19,9 g Pyridinsulfon (125 mmol) zugegeben und der pH-Wert durch Zugabe von 32%iger Natronlauge auf pH-Wert 8 eingestellt. Der Reaktionsansatz wird 12 h unter N₂ gerührt, danach filtriert und das Filtrat unter vermindertem Druck auf 50 g eingeengt (T = 60 °C; p =100 mbar). Zu dem eingeengten Filtrat werden 250 ml Methanol innerhalb von 1 h zugetropft und der ausgefallene Feststoff (Natriumsulfat) abfiltriert. Nach dem Trocknen wird sulfatisiertes Tilirosid, Natriumsalz erhalten.

### Beispiel 1

### Lotion (W/O) zum Auftragen auf die Haut

| | | **Gew.%** |
|---|---|---|
| **A** | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | Tilirosid | 0,5 |
| | | |
| **B** | Glycerin | 5,0 |
| | Magnesiumsulfat-Heptahydrat | 1,0 |
| | Konservierungsmittel | q.s. |
| | Wasser, demineralisiert | ad 100 |

### Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:
0,05 % Propyl-4-hydroxybenzoat
0,15 % Methyl-4-hydroxybenzoat

### Beispiel 2

### Lotion (W/O) zum Auftragen auf die Haut

| | | **Gew.%** |
|---|---|---|
| **A** | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | | |
| **B** | sulfatisiertes Tilirosid, Natriumsalz (Beispiel A) | 1,0 |
| | Glycerin | 5,0 |
| | Magnesiumsulfat-Heptahydrat | 1,0 |
| | Konservierungsmittel | q.s. |
| | Wasser, demineralisiert | ad 100 |

### Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:
0,05 % Propyl-4-hydroxybenzoat
0,15 % Methyl-4-hydroxybenzoat

### Beispiel 3

### Sonnenschutzspray (O/W)

| | | | **Gew.%** |
|---|---|---|---|
| **A** | Eusolex 9020 | (1) | 2,0 |
| | Eusolex^{®}HMS | (1) | 7,0 |
| | Steareth-2 | | 0,4 |
| | Steareth-10 | | 0,8 |
| | Pemulen^{®} TR-2 | (2) | 0,18 |
| | Hetester PHA | (3) | 5,0 |
| | Performa^{®} V 825 | (4) | 0,8 |
| | Dimeticon | | 1,0 |
| | Oxynex^{®}K | (1) | 0,1 |
| | | | |
| **B** | sulfatisiertes Tilirosid, Natriumsalz (Beispiel A) | | 1,0 |
| | Eusolex^{®}232 | (1) | 1,0 |
| | Triethanolamin | (1) | 0,9 |
| | Propandiol-1,2 | (1) | 2,0 |
| | Wasser, demineralisiert | | ad 100 |

### Herstellung

### Phase A

Die Bestandteile der Phase A mit Ausnahme von Permulen^{®}TR-2 werden zusammengegeben und auf 80°C erwärmt. Anschließend wird das Permulen^{®}TR-2 unter Rühren zugegeben.

### Phase B

Das Wasser wird mit dem Triethanolamin vermischt und anschließend Eusolex^{®}232 unter Rühren zugegeben. Sobald sich alles gelöst hat, werden die weiteren Bestandteile der Phase B zugegeben und anschließend die Mischung auf 80°C erwärmt.

### Herstellung des Sonnenschutzmittels

Zur Phase A wird unter Rühren langsam die Phase B gegeben. Nach dem Homogenisieren wird unter Rühren abgekühlt.

Als Konservierungsmittel werden verwendet:
0,05 % Propyl-4-hydroxybenzoat
0,15 % Methyl-4-hydroxybenzoat

### Bezugsquellen

| | |
|---|---|
| (1) | Merck KGaA |
| (2) | BF Goodrich |
| (3) | Bernel |
| (4) | New Phase |

### Beispiel 4

### Sonnenschutzcreme (O/W)

| | | | **Gew.%** |
|---|---|---|---|
| **A** | Eusolex^{®} 2292 | (1) | 4,0 |
| | Eusolex^{®} 9020 | (1) | 1,0 |
| | Eusolex^{®} 6300 | (1) | 1,0 |
| | Stearinsäure | (1) | 2,5 |
| | Imwitor 960 K Schuppen | (2) | 1,0 |
| | Antaron V-220 | (3) | 2,0 |
| | Cetylalkohol | (1) | 0,3 |
| | Miglyol 812 N | (4) | 5,5 |
| | Jojobaöl | (5) | 2,0 |
| | Pemulen TR-1 | (6) | 0,25 |
| | Tilirosid | (1) | 1,0 |
| | | | |
| **B** | Glycerin, wasserfrei | (1) | 3,0 |
| | Propyl-4-hydroxybenzoat | (1) | 0,1 |
| | Methyl-4-hydroxybenzoat | (1) | 0,2 |
| | Keltrol RD | | 0,4 |
| | Wasser, demineralisiert | | ad 100 |
| | | | |
| **C** | Triethanolamin reinst | (1) | 0,9 |
| | | | |
| **D** | SFE 839 | (7) | 5,0 |
| | Arlacel 80 | (8) | 0,3 |

### Herstellung

Phase A wird gemischt und auf 75°C erhitzt. Phase B wird gemischt und auf 70°C erhitzt. Anschliessend wird Phase A zu Phase B gegeben, das Gemisch homogenisiert und unter Rühren auf 45 °C abgekühlt. Danach werden Phase C und Phase D unter Rühren zugegeben.

### Bezugsquellen

| | |
|---|---|
| (1) | Merck KGaA |
| (2) | Condea Chemie GmbH |
| (3) | ISP Global Technologies |
| (4) | Condea Chemie GmbH |
| (5) | Gustav Heess GmbH |
| (6) | BF Goodrich GmbH |
| (7) | GE Silicones Holland |
| (8) | Uniqema |

### Beispiel 5

### Lotion (W/O) zum Auftragen auf die Haut

| | | **Gew.%** |
|---|---|---|
| **A** | 4,6,3',4'-Tetrahydroxybenzylcoumaranon-3 | 1,0 |
| | Polyglyceryl-2-Dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | Tilirosid | 1,0 |
| | | |
| **B** | Glycerin | 5,0 |
| | Magnesiumsulfat-Heptahydrat | 1,0 |
| | Konservierungsmittel | q.s. |
| | Wasser, demineralisiert | ad 100 |

### Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:
0,05 % Propyl-4-hydroxybenzoat
0,15 % Methyl-4-hydroxybenzoat

### Beispiel 6

### O/W After Sun Lotion

| | | | **Gew.%** |
|---|---|---|---|
| **A** | RonaCare^{™} Bisabolol | (1) | 0,3 |
| | Montanov 68 | (2) | 4,0 |
| | Miglyol 812, Neutralöl | (3) | 12,0 |
| | Mirasil CM5 | (4) | 2,0 |
| | Mirasil DM 350 | (4) | 1,0 |
| | Tilirosid | | 1,0 |
| | | | |
| **B** | Wasser, demineralisiert | | ad 100 |
| | Glycerin (87% reinst) | (1) | 3,0 |
| | Konservierungsmittel | | q.s. |
| | RonaCare^{™} Ectoin | (1) | 1,0 |
| | | | |
| **C** | Rhodicare S | (4) | 0,5 |

### Herstellung

Phasen A und B werden getrennt auf 75°C erhitzt. Phase C wird bei 75°C unter Rühren langsam zu Phase B zugegeben und es wird gerührt, bis eine homogene Mischung entsteht. Anschließend wird Phase A zu der Mischung B/C gegeben und homogenisiert. Unter Rühren wird die erhaltene Mischung auf Raumtemperatur abgekühlt.

Als Konservierungsmittel werden verwendet:
0,05 % Propyl-4-hydroxybenzoat
0,15 % Methyl-4-hydroxybenzoat

| Bezugsquellen | |
|---|---|
| (1) | Merck KGaA |
| (2) | Seppic |
| (3) | Condea Chemie GmbH |
| (4) | Rhodia GmbH |

### Beispiel 7

### Sonnenschutzlotion (W/O)

| | | | **Gew.%** |
|---|---|---|---|
| **A** | Eusolex 8300 | (1) | 4,0 |
| | Eusolex 2292 | (1) | 7,0 |
| | Abil WE 09 | (2) | 5,0 |
| | Jojobaöl | (3) | 3,0 |
| | Cetiol V | (4) | 3,0 |
| | Prisorine 2021 | (5) | 2,0 |
| | Paracera M | | 1,0 |
| | Miglyol 812, Neutralöl | (6) | 3,0 |
| | Propyl-4-hydroxybenzoat | (1) | 0,05 |
| | Tilirosid | | 1,0 |
| | | | |
| **B** | Eusolex T-Aqua | (1) | 16,0 |
| | Glycerin (87% reinst) | (1) | 2,0 |
| | Natriumchlorid | (1) | 0,4 |
| | RonaCare^{™} Ectoin | (1) | 1,0 |
| | Wasser, demineralisiert | | ad 100 |
| | Methyl-4-hydroxybenzoat | (1) | 0,15 |

### Herstellung

Phase B wird auf 80°C und Phase A wird auf 75°C erhitzt. Phase B wird langsam in Phase A eingerührt. Das Gemisch wird homogenisiert und unter Rühren abgekühlt.

### Bezugsquellen

(1) Merck KGaA
(2) Goldschmidt AG
(3) Gustav Heess GmbH
(4) Cognis GmbH
(5) Uniqema
(6) Condea Chemie GmbH

### Beispiel 8

Aus folgenden Komponenten wird eine Creme (O/W), enthaltend Ectoin, hergestellt:

| | | | **Gew.%** |
|---|---|---|---|
| **A** | Paraffin, dünnflüssig | (1) | 8,0 |
| | Isopropylmyristat | (1) | 4,0 |
| | Mirasil CM5 | (2) | 3,0 |
| | Stearinsäure | (1) | 3,0 |
| | Arlacel 165 V | (3) | 5,0 |
| | Tilirosid | | 1,0 |
| | | | |
| **B** | Glycerin (87%) | (1) | 3,0 |
| | Germaben ll | (4) | 0,5 |
| | Wasser, demineralisiert | | ad 100 |
| | C RonaCare^{™} Ectoin | (1) | 1,0 |

### Herstellung

Zunächst werden die Phasen A und B getrennt auf 75°C erwärmt. Danach wird Phase A unter Rühren langsam zu Phase B gegeben und solange gerührt, bis eine homogene Mischung entsteht. Nach Homogenisierung der Emulsion wird unter Rühren auf 30°C abgekühlt. Anschließend wird auf 35°C erwärmt, die Phase C zugegeben und bis zur Homogenität gerührt.

### Bezugsquellen

| | |
|---|---|
| (1) | Merck KGaA |
| (2) | Rhodia |
| (3) | Uniqema |
| (4) | ISP |

### Beispiel 9

### Topische Zusammensetzung als W/O-Emulsion

| | | | **Gew.%** |
|---|---|---|---|
| **A** | Isolan PDI | (2) | 3,0 |
| | Paraffinöl, fl. | (1) | 17,0 |
| | Isopropylmyristat | | 5,0 |
| | Bienenwachs | | 0,2 |
| | Cutina HR | (2) | 0,3 |
| | Tilirosid | | 1,0 |
| | | | |
| **B** | Wasser, demineralisiert | | ad 100 |
| | Glycerin (87%) | | 4,0 |
| | Magnesiumsulfat | | 1,0 |
| | Germaben II-E | (3) | 1,0 |
| | | | |
| **C** | RonaCare^{™} LPO | (1) | 2,0 |

### Herstellung

Die Phasen A und B werden auf 75°C erwärmt. Phase B wird unter Rühren zu Phase A gegeben. Anschließend wird das Gemisch bei 9000upm 2 Min. mit dem Turrax homogenisiert. Das erhaltene Gemisch wird auf 30 bis 35°C abgekühlt, und C wird eingerührt.

### Bezugsquellen

(1) Merck KGaA
(2) Goldschmidt AG
(3) ISP

### Beispiel 10

### After-Sun-Lotion (O/W)

| | | | **Gew.%** |
|---|---|---|---|
| **A** | Biobase^{™} EP | (4) | 4,5 |
| | Isopropylpalmitat | (1) | 3,0 |
| | Cetiol V | (1) | 2,5 |
| | Miglyol 812 | (2) | 9,0 |
| | Carbopol ETD 2050 | (3) | 0,3 |
| | RonaCare^{™} LPO | (2) | 1,0 |
| | Tilirosid | | 1,0 |
| | | | |
| **B** | Wasser, demineralisiert | | ad 100 |
| | Glycerin (87% reinst) | (2) | 3,0 |
| | Konservierungsmittel | | q.s. |
| | | | |
| **C** | Natronlauge, 10%ig | (2) | |

### Herstellung

Die Phasen A und B werden getrennt auf 70°C erwärmt. Anschliessend wird Phase B unter Rühren zu Phase A gegeben. Danach wird das Gemisch homogenisiert, mit Natronlauge neutralisiert und unter Rühren abgekühlt.

Als Konservierungsmittel werden verwendet:
0,05 % Propyl-4-hydroxybenzoat
0,15 % Methyl-4-hydroxybenzoat

### Bezugsquellen

| | |
|---|---|
| (1) | Cognis GmbH |
| (2) | Merck KGaA |
| (3) | BF Goodrich GmbH |
| (4) | Tri-K Industries, Inc. |

### Beispiel 11

### Hautpflegegel (O/W)

| | | | **Gew.%** |
|---|---|---|---|
| **A** | Eusolex^{®} 6300 | (1) | 1,0 |
| | RonaCare^{™} Tocopherolacetat | (1) | 1,0 |
| | Avocadoöl | (2) | 5,0 |
| | Jojobaöl | (2) | 5,0 |
| | Miglyol 812 N | (3) | 3,0 |
| | Eutanol G | (4) | 5,0 |
| | Sepigel 305 | (5) | 3,0 |
| | Tilirosid | (1) | 0,5 |
| | | | |
| **B** | Wasser, demineralisiert | | ad 100 |
| | Karion F flüssig | (1) | 5,0 |
| | Panthenol-D | (6) | 1,0 |
| | | | |
| **C** | RonaCare^{™} ASC III | (1) | 4,0 |
| | Parfümöl TND-2417 | (7) | 0,1 |
| | Konservierungsmittel | | q.s. |

### Herstellung

Die Phasen A und B werden separat vorgelöst. Anschliessend wird Phase B unter Rühren zu Phase A gegeben und nach und nach die Bestandteile der Phase C zugegefügt.

Als Konservierungsmittel werden verwendet:
0,05 % Propyl-4-hydroxybenzoat
0,15 % Methyl-4-hydroxybenzoat

### Bezugsquellen

| | |
|---|---|
| (1) | Merck KGaA |
| (2) | Gustav Heess GmbH |
| (3) | Condea Chemie GmbH |
| (4) | Cognis GmbH |
| (5) | Interogana GmbH |
| (6) | Hoffmann-La Roche AG |
| (7) | Takasago |

## Patentansprüche

1. Verwendung von einer oder mehreren Verbindungen der Formel IA, 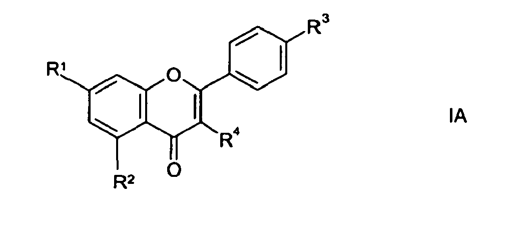 worin
R¹, R² und R³ jeweils unabhängig voneinander OH, CH₃COO, einen Alkoxyrest mit 1 bis 8 C-Atomen oder einen Monoglykosidrest bedeuten,
R⁴ ein Mono- oder Diglykosidrest ist, wobei an den Glykosidrest jeweils über eine Gruppe -O- 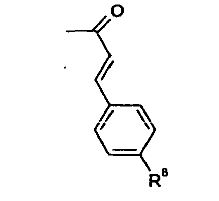 gebunden ist,
R⁸ die Bedeutung der Reste R¹ bis R³ besitzt, und
worin ein oder mehrere Wasserstoffatome in den OH-Gruppen des oder der Glykosidreste jeweils unabhängig voneinander auch durch Acetyl oder durch Alkylreste mit 1 bis 8 C-Atomen ersetzt sein können und wobei an ein oder mehrere Hydroxygruppen der Verbindungen der Formel IA jeweils unabhängig voneinander auch Sulfat oder Phosphat gebunden sein kann, wobei die eine oder mehreren Verbindungen der Formel IA in Form einer synthetisch gewonnenen Substanz oder in Form der aus einem Pflanzenextrakt gewonnenen Reinsubstanz vorliegen, zur Herstellung von kosmetischen Formulierungen.

2. Verwendung von einer oder mehreren Verbindungen der Formel IA aus Anspruch 1, wobei die eine oder mehreren Verbindungen der Formel IA in Form einer synthetisch gewonnenen Substanz oder in Form der aus einem Pflanzenextrakt gewonnenen Reinsubstanz voliegen, als Aktivstoff in kosmetischen Formulierungen.

3. Verwendung von einer oder mehreren Verbindungen der Formel IA aus Anspruch 1 als UV-Filter in kosmetischen Formulierungen.

4. Verwendung von einer oder mehreren Verbindungen der Formel IA aus Anspruch 1 als Radikalfänger und/oder Antioxidans in kosmetischen Formulierungen.

5. Verwendung von einer oder mehreren Verbindungen der Formel IA aus Anspruch 1 gegen oxidativen Stress in kosmetischen Formulierungen.

6. Verwendung von einer oder mehreren Verbindungen der Formel IA aus Anspruch 1 zur Vermeidung der Hautalterung in kosmetischen Formulierungen.

7. Verwendung von einer oder mehreren Verbindungen der Formel IA aus Anspruch 1 als Wirkstoff mit anti-allergischer, anti-inflammatorischer, entzündungshemmender oder anti-irritativer Wirkung in kosmetischen Formulierungen.

8. Verwendung von einer oder mehreren Verbindungen der Formel IA aus Anspruch 1 zur Stabilisierung von UV-Filtern, insbesondere Dibenzoylmethan und Derivaten des Dibenzoylmethans in kosmetischen Formulierungen.

9. Verwendung nach einem oder mehreren der Ansprüche 1 bis 8, **dadurch gekennzeichnet, dass** die Verbindung der Formel IA Tilirosid ist.

10. Tilirosid, **dadurch gekennzeichnet, dass** an eine oder an mehrere Hydroxygruppen Sulfat gebunden ist.

## Claims

1. Use of one or more compounds of the formula IA 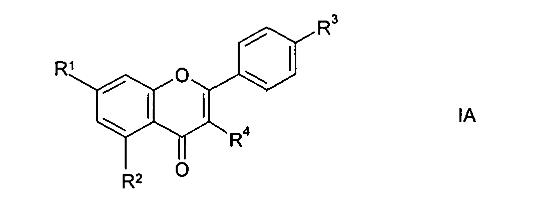 in which
R¹, R² and R³ each, independently of one another, denote OH, CH₃COO, an alkoxy radical having 1 to 8 C atoms or a monoglycoside radical,
R⁴ denotes a mono- or diglycoside radical, where 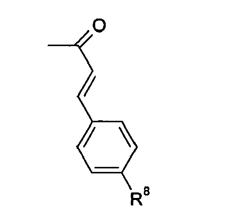 is bonded to the glycoside radical in each case via a group -O-,
R⁸ has the meaning of the radicals R¹ to R³, and
in which one or more hydrogen atoms in the OH groups of the glycoside radical(s) may each, independently of one another, also be replaced by acetyl or by alkyl radicals having 1 to 8 C atoms, and where, in each case independently of one another, sulfate or phosphate may also be bonded to one or more hydroxyl groups of the compounds of the formula IA, where the one or more compounds of the formula IA are in the form of a substance obtained synthetically or in the form of the pure substance obtained from a plant extract, for the preparation of cosmetic formulations.

2. Use of one or more compounds of the formula IA from Claim 1, where the one or more compounds of the formula IA are in the form of a substance obtained synthetically or in the form of the pure substance obtained from a plant extract, as active substance in cosmetic formulations.

3. Use of one or more compounds of the formula IA from Claim 1 as UV filter in cosmetic formulations.

4. Use of one or more compounds of the formula IA from Claim 1 as free-radical scavenger and/or antioxidant in cosmetic formulations.

5. Use of one or more compounds of the formula IA from Claim 1 against oxidative stress in cosmetic formulations.

6. Use of one or more compounds of the formula IA from Claim 1 for preventing skin ageing in cosmetic formulations.

7. Use of one or more compounds of the formula IA from Claim 1 as active compound having an anti-allergic, anti-inflammatory, inflammation-inhibiting or anti-irritative action in cosmetic formulations.

8. Use of one or more compounds of the formula IA from Claim 1 for the stabilisation of UV filters, in particular dibenzoylmethane and derivatives of dibenzoylmethane, in cosmetic formulations.

9. Use according to one or more of Claims 1 to 8, **characterised in that** the compound of the formula IA is tiliroside.

10. Tiliroside, **characterised in that** sulfate is bonded to one or more hydroxyl groups.

## Revendications

1. Utilisation d'un ou plusieurs composés de formule IA 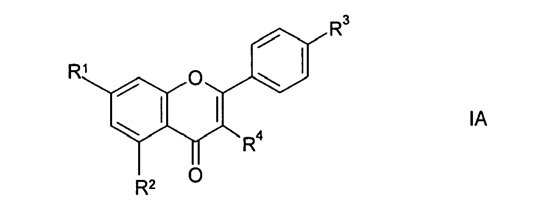 dans laquelle
R¹, R² et R³ désignent chacun, indépendamment les uns des autres, OH, CH₃COO, un radical alcoxy ayant de 1 à 8 atomes de C ou un radical monoglycoside,
R⁴ désigne un radical mono- ou diglycoside, où 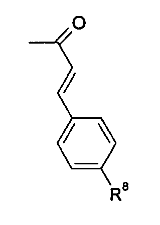 est lié au radical glycoside dans chaque cas par l'intermédiaire d'un groupement -O-,
R⁸ a la signification des radicaux R¹ à R³, et
dans laquelle un ou plusieurs atomes d'hydrogène dans les groupements OH du ou des radicaux glycoside peuvent chacun, indépendamment les uns des autres, être également remplacés par acétyle ou par des radicaux alkyle ayant de 1 à 8 atomes de C, et où, dans chaque cas indépendamment les uns des autres, du sulfate ou du phosphate peut également être lié à un ou plusieurs groupements hydroxyle des composés de formule lA, où lesdits un ou plusieurs composés de formule IA sont sous la forme d'une substance obtenue synthétiquement ou sous la forme de la substance pure obtenue à partir d'un extrait végétal, pour la préparation de formulations cosmétiques.

2. Utilisation d'un ou plusieurs composés de formule IA selon la revendication 1, où lesdits un ou plusieurs composés de formule lA sont sous la forme d'une substance obtenue synthétiquement ou sous la forme de la substance pure obtenue à partir d'un extrait végétal, comme substance active dans des formulations cosmétiques.

3. Utilisation d'un ou plusieurs composés de formule IA selon la revendication 1, comme filtre UV dans des formulations cosmétiques.

4. Utilisation d'un ou plusieurs composés de formule IA selon la revendication 1, comme agent anti-radicalaire et/ou antioxydant dans des formulations cosmétiques.

5. Utilisation d'un ou plusieurs composés de formule IA selon la revendication 1, contre le stress oxydatif dans des formulations cosmétiques.

6. Utilisation d'un ou plusieurs composés de formule IA selon la revendication 1, pour la prévention du vieillissement cutané dans des formulations cosmétiques.

7. Utilisation d'un ou plusieurs composés de formule IA selon la revendication 1, comme composé actif ayant une action antiallergique, anti-inflammatoire, inhibitrice d'inflammations ou anti-irritante dans des formulations cosmétiques.

8. Utilisation d'un ou plusieurs composés de formule IA selon la revendication 1, pour la stabilisation de filtres UV, en particulier le dibenzoylméthane et les dérivés de dibenzoylméthane, dans des formulations cosmétiques.

9. Utilisation selon une ou plusieurs parmi les revendications 1 à 8, **caractérisée en ce que** le composé de formule IA est le tiliroside.

10. Tiliroside, **caractérisé en ce que** du sulfate est lié à un ou plusieurs groupements hydroxyle.
